# EUROPEAN PATENT APPLICATION

(11) **EP 4 560 028 A1**
(43) Date of publication of application: **28.05.2025**
(21) Application number: 23841703.4
(22) Date of filing: 02.02.2023
(51) Int. Cl.: C12Q 1/6874, C12N 15/11, C12Q 1/6834

(54) **RAPID NEXT-GENERATION SEQUENCING METHOD**

(30) Priority: 21.07.2022 CN 202210866435
(71) Applicant: Shenzhen Salus Biomed Co., Ltd, Shenzhen, Guangdong 518107 (CN)
(72) Inventor: ZHANG, Changling, Shenzhen, Guangdong 518107 (CN); WANG, Gufeng, Shenzhen, Guangdong 518107 (CN); ZHAO, Luyang, Shenzhen, Guangdong 518107 (CN)
(74) Representative: Cabinet Chaillot
(86) International application number: PCT/CN2023/074257
(87) International publication number: WO 2024/016625

(57) **Abstract**

The present application provides a rapid next-generation gene sequencing method, which includes the following steps: incorporation: combining a detection complex to a primer hybridized to a nucleic acid molecule to be detected, wherein the detection complex is modified with a fluorescence group and a blocker group; scanning: exciting the fluorescence group to generate a fluorescence signal, obtaining an image signal through an optical imaging system, and identifying the image signal to obtain the type of a base; cleavage: removing the fluorescence group and the blocker group; repeating the cycle of the incorporation-scanning-cleavage; wherein the scanning is started after the beginning of the incorporation step, and/or the cleavage is started after the beginning of the scanning step. In the sequencing method of the present application, unfinished incorporation reaction and preparatory work for the cleavage reaction are performed during the signal scanning period which takes a long period of time, which reduces the time of a single cycle, thereby finally shortening the time for the entire sequence process.

## Description

### CROSS-REFERENCE

The present application claims the priority of China Patent Application No. 202210866435.9, entitled "RAPID NEXT-GENERATION GENE SEQUENCING METHOD", and filed on July 21, 2022 to China Patent Office; the whole content thereof is incorporated herein by reference.

### FIELD

The present application relates to the field of gene sequencing, and more particularly, relates to a rapid next-generation gene sequencing method.

### BACKGROUND

In order to overcome the drawbacks of high cost and low throughput of the first-generation gene sequencing, a next-generation sequencing (NGS) is developed accordingly, which is based on the principle of sequencing by synthesis (SBS). Representative technology of the presently available next-generation sequencing technology is the bridge amplification technology developed by Illunina, Inc. and DNBSEQ technology developed by BGI Genomics, which realizes the identification and distinction of the four bases (A, C, G and T/U) in gene sequence by detecting the fluorescence signals. Specifically, these methods use the dNTP (dATP, dCTP, dGTP, dTTP) which carries fluorescence marker and blocker groups and the fluorescence marker groups carried by the dATP, dCTP, dGTP and the dTTP are different from each other. Due to the existence of the blocker groups, during each cycle of reaction of DNA incorporation, each DNA template is added with only one complementary dNTP. The type of the dNPT added in this cycle can be identified by using laser having the corresponding spectrum to excite the added dNTP. Then a suitable chemical reagent is used to cut off the blocker groups and the fluorescence marker groups whereby the sequencing reaction can normally enter the next cycle. However, the cycle of the presently available sequencing process is relatively long. What is needed is to provide a sequencing method which can significantly shorten the sequencing period of time with the premise that the sequencing quality is guaranteed.

### SUMMARY

An object of the present application is to overcome at least one of the above-mentioned problems in the prior art. To achieve this, the present application proposes a rapid next-generation gene sequencing method, through which the sequencing period of time can be effectively shortened with the premise that the sequencing quality is guaranteed.

According to one aspect of the present application, a sequencing method is provided which includes the following steps:
Incorporation: combining a detection complex to a primer hybridized to a nucleic acid molecule to be detected, wherein the detection complex is modified with a fluorescence group and a blocker group;
Scanning: exciting the fluorescence group to generate a fluorescence signal, and identifying the fluorescence signal to obtain the type of base;
Cleavage: removing the fluorescence group and the blocker group;
Repeating the cycle of incorportation-scannning-cleavage;
The scanning is started after the beginning of the incorporation step, and/or the cleavage is started after the beginning of the scanning step.

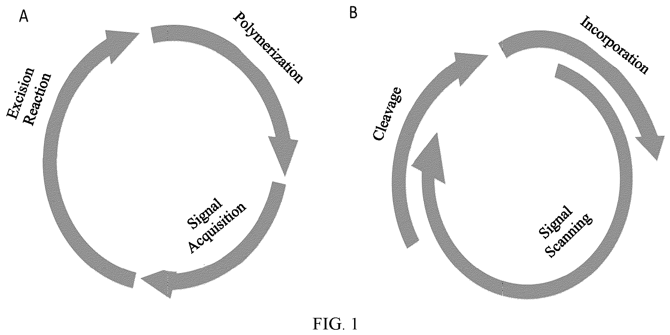

The sequencing method in accordance with the present application has at least the following advantages:
Referring to A of FIG. 1, in the presently available general next-generation sequencing process, an incorporation reaction is first performed, and after the completion of the incorporation reaction, a signal scanning is conducted. And the cleavage reaction is conducted after the completion of the signal scanning; then the next cycle is entered. However, in the present application, referring to B of FIG. 1, to sufficiently utilize the period of time during which the signal is scanned, at least a part of the polymerization and cleavage process is conducted in this stage. After the detection complex is partially fed into the reaction system and is combined to the primer, the signal scanning process is started; and/or, during the process of the signal scanning, a pretreatment of the cleavage process is started for the nucleic acid molecule to be detected at a specific position where the signal scanning has been completed. Thus, unfinished incorporation reaction and preparatory work for the cleavage reaction are performed during the signal scanning period which takes a long period of time, thereby reducing the time of a single cycle, and finally significantly shortening the time for the entire sequence process.

It can be understood that the above-mentioned and hereafter-mentioned "start after a specific step" generally means that the start is after the beginning and before the completion of the specific step. In respect to the above-mentioned method, it means that the scanning is started before the completion of the incorporation step, and/or the cleavage is started before the completion of the scanning step.

The detection complex means a substance including any reversible terminator adopted according to the principle of sequencing by synthesis (SBS), which can realize the addition to and identification of a single base. The specific examples include but not limited to deoxyribonucleotide individually modified with blocker group and fluorescence group. In this manner, scars will generate, which accumulatively affects the efficiency of incorporation and cleavage. To resolve this problem, a complex including a plurality of deoxyribonucleotides respectively modified with blocker group and fluorescence group can be used. Or, the deoxyribonucleotide is compounded with other substances to form a complex modified with fluorescence group and blocker group, etc. The specific examples of the fluorescence group include but not limited to FAM, HEX, TAMRA, Cy3, Cy3.5, Cy5, Cy5.5., Cy7, Texas, ROX, JOE, R6G, EDANS, IFlour, Alexa Flour, FITC, etc. The specific examples of the blocker group include but not limited to azidomethyl-group, amino-group, allyl-group, phosphate-group, 3'-O-(2-cyanoethyl-group), etc.

In some embodiments of the present application, the step of linking a detection complex to a primer hybridized to a nucleic acid molecule to be detected, includes:
Feeding the detection complex and a polymerase onto a surface of a solid phase carrier, the surface being loaded with the nucleic acid molecule to be detected, and the primer being hybridized to the nucleic acid molecule to be detected;
Under the action of the polymerase, the detection complex is combined to the primer and a blockage occurs;
The polymerase can be a wild or mutated polymerase having a source originated from bacteria. To ensure that the reversible terminator in the detection complex can be combined to the primer with high efficiency, a polymerase having a high capability of incorporation into the substrate can be selected, such as polymerase 9N, etc.

In some embodiments of the present application, the detection complex includes a first nucleotide analog, and the step of combining a detection complex to a primer hybridized to a nucleic acid molecule to be detected, includes:
Feeding the first nucleotide analog and the polymerase onto the surface of the solid phase carrier;
Under the action of the polymerase, the first nucleotide analog is combined to the primer and a blockage occurs to thereby form the detection complex.

In some embodiments of the present application, the scanning is started after the beginning of the step of feeding the first nucleotide analogue and the polymerase onto the surface of the solid phase carrier.

In some embodiments of the present application, after the formation of the detection complex, the step of incorporation further includes:
Feeds a second nucleotide analogue and the polymerase onto the surface of the solid phase carrier;
Under the action of the polymerase, the second nucleotide analog is combined to the primer which has not been combined with the first nucleotide analog, and a blockage occurs. During the process that the first nucleotide analog is introduced to a large amount of the primer strands, it is possible that some primers have not been timely introduced with the first nucleotide analog whereby a problem may happen that the scanning of the base signal can be missed in one of the cycles. The addition of the second nucleotide analog can make the incorporation and scanning in every cycle more complete, to thereby prevent the next cycle from being affected by the insufficient reaction of the present cycle.

In addition, since the second nucleotide analog merely acts to make up for the deficiency that some primers fail to be combined with the first nucleotide analog and does not affect the combining between the first nucleotide analogue itself and the primer and the fluorescence signal scanning. Therefore, the step of signal scanning can be conducted concurrently with the feeding of the second nucleotide analog and the polymerase onto the surface of the solid phase carrier, or even can be conducted after the completion of the feeding of the first nucleotide analog and the polymerase onto the surface of the solid phase carrier, or in a set period of time (for example, 0 s-N s) after the beginning of feeding the first nucleotide analog and the polymerase onto the surface of the solid phase carrier, wherein n for example can be 60s, 90s, 120s, 180s, etc. As long as a reagent is added such that at least a portion of the primer has already been combined with the first nucleotide analog and the detection of the fluorescence signal is made possible, the step of signal scanning can be started.

In some embodiments of the present application, the second nucleotide analog is modified with a blocker group. Further, the second nucleotide analog is only modified with the blocker group. For example, at the 3-hydroxy of the ribose of the second nucleotide analogue, it is modified with the blocker group.

In some embodiments of the present application, the first nucleotide analog is modified with the fluorescence group and the blocker group. For example, at the base of the first nucleotide analog, it is modified with the fluorescence group, and at the 3-hydroxy of the ribose thereof, it is modified with the blocker group.

In some embodiments of the present application, the scanning is started after the beginning of the step of feeding the second nucleotide analog onto the surface of the solid phase carrier.

In some embodiments of the present application, the solid phase carrier can be a flowcell. It can be understood that the solid phase carrier can be any other carrier which can provide an environment for incorporation and severance reaction.

In some embodiments of the present application, the step of removing the blocker group and the fluorescence group includes:
Feeding cleavage agent onto the surface of the solid phase carrier to remove the blocker group and the fluorescence group in the detection complex.

In some embodiments of the present application, before the cleavage, an elution reagent is fed onto the surface of the solid phase carrier to remove the residual reagent from the surface.

In some embodiments of the present application, the step of feeding the elution reagent onto the surface of the solid phase carrier to remove the residual reagent from the surface is started after the beginning of the step of scanning.

In some embodiments of the present application, the start of the scanning after the beginning of the step of incorporation means that the scanning is started in 0-60s after the beginning of the step of incorporation. In this manner, the signal scanning is started during the process of the incorporation reaction; thus, as soon as the completion of incorporation of a portion of DNA amplification cluster, the signal scanning is immediately conducted. Accordingly, the delay of time in the early stage due to that the signal scanning is started after all of the incorporation reactions have been completed can be avoided. For example, the signal scanning can be started in 1s, 2s, 3s, 5s, 10s, 15s, 20s, 25s, 30s, 35s, 40s, 45s, 50s, 55s, 60s after the beginning of the step of incorporation.

In some embodiments of the present application, the start of cleavage after the beginning of the step of scanning means that the cleavage is started in 0-10min after the beginning of the step of scanning. For the portion of the DNA amplification cluster whose base signal has been scanned, the process of cleavage reaction or pretreatment thereof can be started before the completion of scanning of all signals. Thus, the delay of time in the later stage due to that the cleavage is started after all of the signal scanning has been completed can be avoided. For example, the cleavage reaction can be started in 0s, 10s, 15s, 30s, 45s, 60s, 2min, 3min, 5min, 8min after the beginning of the step of scanning. According to the required period of time of different signal collection systems and the liquid feeding speed of the subsequent cleavage reagent, the cleavage reaction can be started after the beginning of the step of scanning.

In some embodiments of the present application, the start of the scanning reaction means first feeding washing buffer for the cleavage reaction, such as elution reagent, onto the surface of the solid phase carrier. Thus, the residue of other reagents can be washed away from the surface of the solid phase carrier to thereby provide a suitable reaction condition for the subsequent cleavage reaction.

In some embodiments of the present application, the sequencing method includes the following steps:

### Incorporation:

Feeding pre-wash buffer onto the surface of the solid phase carrier to thereby wash away the possible residual reagent of the process of library construction, amplification, etc;
Feeding incorporation reaction solution 1 onto a surface of a solid phase carrier, the surface being fixed thereon with nuclei acid molecule to be detected, the incorporation reaction solution 1 including a first nucleotide analog, polymerase, etc, the first nucleotide analog being modified with the blocker group and the fluorescence group, at a set temperature and under the action of the polymerase, etc, the first nucleotide analog being combined to the primer and a blockage occurring;
Feeding incorporation reaction solution 2 onto the surface of the solid phase carrier, the incorporation reaction solution 2 including a second nucleotide analog, polymerase, etc, the second nucleotide analog being modified with the blocker group, at a set temperature and under the action of the polymerase, etc, the second nucleotide analog being combined to the primer which has not been combined with the first nucleotide analog and a blockage occurring;

### Scanning:

Exciting the fluorescence group to generate the fluorescence signal, identifying the fluorescence signal to acquire the type of the base;

### Cleavage:

Feeding an elution buffer onto the surface of the solid phase carrier to remove the residual reagent;
Feeding cleavage reaction solution onto the surface of the solid phase carrier, the cleavage reaction solution including a reagent which can separately or simultaneously remove the blocker group and the fluorescence group from the detection complex or the first nucleotide analog, the cleavage reaction solution reacting under a set temperature, and then refeeding the elution buffer to remove the residual reagent after the cleavage.

Repeating the above cycle until the sequencing is completed.

The step of scanning is started before the completion of the step of incorporation, and/or the step of cleavage is started before the completion of the step of scanning.

It can be understood that the specific composition of the pre-wash buffer, the elution buffer, etc. belongs to the common composition in the art and, therefore, details thereof are omitted here.

It can be understood that, based on the above concept, the detection complex can also be another detection agent having the nucleotide analogs for next-generation DNA sequencing known by those skilled in the art. For example, the detection complex includes a second nucleotide analog and a second detection molecular. The second molecular includes at least one of a third nucleotide analog and a specific antibody of the first nucleotide analog. For example, the step of incorporation includes introducing the second nucleotide analog having the blocker group to the primer strand to form the initial blocking. The third nucleotide analog, which is modified with the fluorescence group, is then bound to the second nucleotide analog through the intermolecular force (for example, at least one of the electrostatic force, hydrogen bond, van der Waals force, hydrophobic force) to form the detection complex for conducting the signal scanning. During this process, the scanning can be started after the step of incorporation. For example, after at least a portion of the third nucleotide analog is bound to the second nucleotide analog to form the detection complex having the fluorescence group, the step of scanning is started, and the fluorescence group is excited to generate the fluorescence signal. In the above-mentioned method, the third nucleotide analog can be replaced by, for example, a specific antibody of the second nucleotide analog. The specific antibody can be specifically bound to the second nucleotide analog of a different base type, and is modified with a different fluorescence group. Thus, the incorporation step includes introducing the second nucleotide analog having the blocker group to the primer strand to form the initial blocking. The specific antibody of the second nucleotide analogue, which is modified with the fluorescence group, is then bound to the second nucleotide analog through the combination of antigens and antibodies, to form the detection complex for conducting the signal scanning. During this process, the scanning can be started after the incorporation step. For example, after at least a portion of the specific antibody is bound to the second nucleotide analog to form the detection complex having the fluorescence group, the step of scanning is started, and the fluorescence group is excited to generate the fluorescence signal.

In some embodiments of the present application, since there is no need for separate signal scanning reagent, the reagent type of the reagent kit for the sequencing can be reduced thereby lowering the sequencing cost.

Based on the above two points, in the actual sequencing process, depending on the factors of the incorporation efficiency of the polymerase used, the reaction efficiency of the incorporation agent used, the reaction solution fed in for incorporation, the liquid delivery efficiency of the pumping system for the buffer, the efficiency of the optical imaging system for obtaining the fluorescence signal images and identifying the type of the bases, the overlapping relationships between the scanning process and the incorporation and cleavage reactions may vary. In some specific embodiments, the overlapping period of time can be extended as long as possible, to thereby shorten the period of time needed for other processing other than the fixed period of time for signal scanning.

In summary, compared with the conventional sequencing process in which the liquid is not pumped in during signal scanning, in the sequencing method provided by the embodiment of the present application, the liquid pumping process for the incorporation and/or cleavage reaction is also conducted during the signal scanning process. Thus, the signal capture is started during the step of the sequencing synthesis reaction. Moreover, during the process of signal capture, the subsequent cleavage can also be started. In addition, in some of the sequencing methods, the whole sequencing process can be performed in a condition having a less temperature fluctuation range, thereby reducing the time required for raising and lowering the temperature. By the above two manners, the period of time required for the sequencing can be shortened.

### BRIEF DESCRIPTION OF THE DRAWINGS

FIG. 1 shows flow charts of the embodiment of the present application and the conventional normal sequencing method.

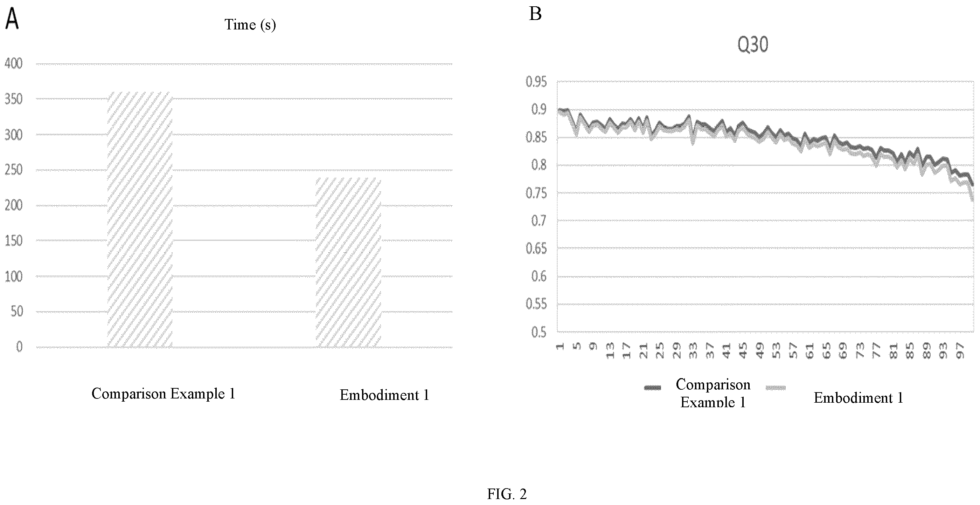

FIG. 2 shows a comparison between the embodiment 1 of the present application and comparison example 1 in terms of the period of time for a single cycle and the sequencing quality for different numbers of cycles, wherein A shows the period of time for a single cycle for the embodiment 1 and the comparison example 1, exclusive of the period of time for signal scanning, and B shows the values of the sequencing quality Q30 of the embodiment 1 and the comparison example 1 for different numbers (1-100) of cycles (abscissa).

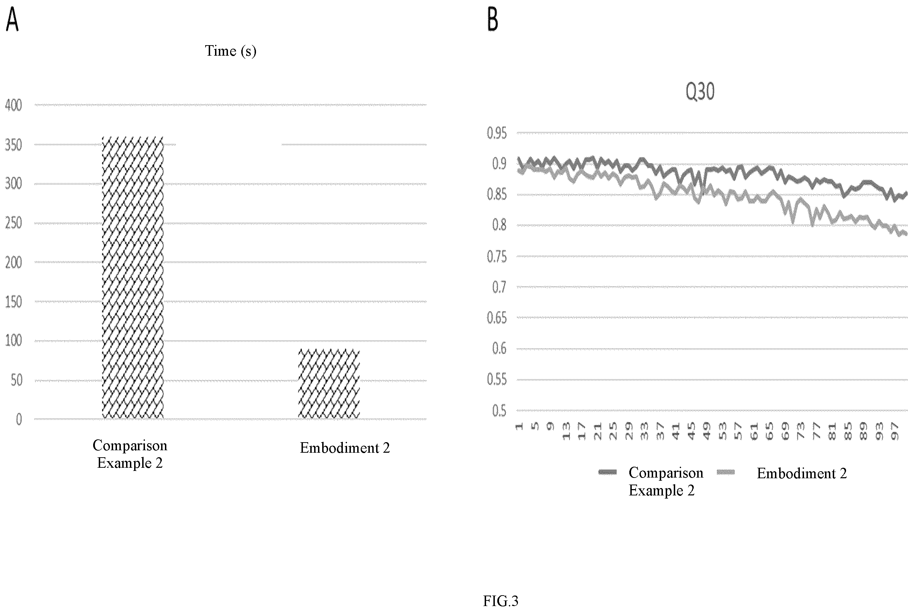

FIG. 3 shows a comparison between the embodiment 2 of the present application and comparison example 2 in terms of the period of time for a single cycle and the sequencing quality for different numbers of cycles, wherein A shows the period of time for a single cycle for the embodiment 2 and the comparison example 2, exclusive of the period of time for signal scanning, and B shows the values of the sequencing quality Q30 of the embodiment 2 and the comparison example 2 for different numbers (1-100) of cycles (abscissa)..

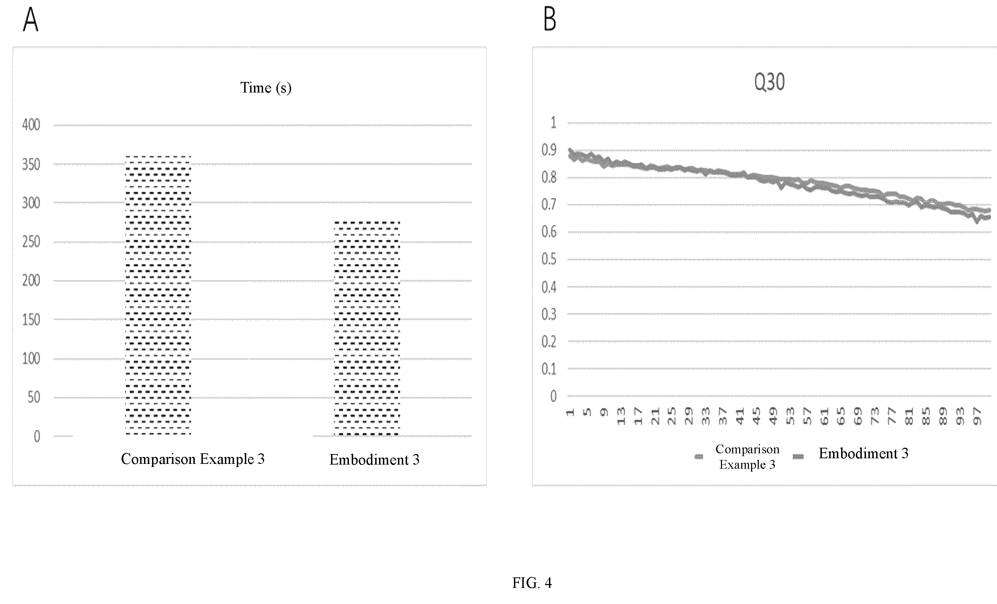

FIG. 4 shows a comparison between the embodiment 3 of the present application and comparison example 3 in terms of the period of time for a single cycle and the sequencing quality for different numbers of cycles, wherein A shows the period of time for a single cycle for the embodiment 3 and the comparison example 3, exclusive of the period of time for signal scanning, and B shows the values of the sequencing quality Q30 of the embodiment 3 and the comparison example 3 for different numbers (1-100) of cycles (abscissa)..

### DESCRIPTION OF THE EMBODIMENTS

For facilitating full understanding of the purposes, technical features, and effects of the present application, a clear and detailed description of the idea of the present application and its accompanying technical effects is provided in combination with the embodiments. It is understood that the embodiments as disclosed are a portion of the embodiments of the present application, not all embodiments. Based on the embodiments of the present application, other embodiments which can be obtained by those skilled in the art without expending creative efforts are all within the scope of the present application.

The following is a detailed description of the embodiments of the present application. The described embodiments are illustrative, used for only explaining the present application, rather than restrictive.

In the description of the present application, "several" means one or more. "Plurality" means two or more. "Larger than", "less than" "exceeding" means that the number specified is exclusive, "above", "under" and "within" means that the number specified is inclusive. If "first", "second" are mentioned, they are used for the purpose of distinguishing the technical features, rather than being understood to indicate or hint at the relative importance, or implicitly indicate the amount of the listed technical features, or implicitly indicate the sequence relation of the listed technical features. In the following description, although the flow chart shows the logic sequence, in some conditions, the shown or described steps can be performed in a sequence different from that of the flow chart.

Except otherwise defined, all of the technological and scientific terminologies used herein are similar to those which can be generally comprehended by those skilled in the art. The terminologies used herein are only for describing the embodiments of the present application, rather than limiting the present application.

In the description of the present application, the terminologies "one embodiment", "some embodiments", "exemplified embodiments", "examples", "specific examples" or "some examples" mean that the specific characteristic, structure, material or feature described in connection with the embodiment or example is included in at least one of the embodiments or examples. In this specification, the illustrative expressions of the above terminologies do not necessarily mean that they are the same embodiments or examples. In addition, the described specific characteristic, structure, material or feature can be combined in a suitable manner in any one or a plurality of the embodiments or examples.

### Embodiment 1

This embodiment provides a sequencing method for human genome, including the following steps:

### 1. Construction of sequencing library

(1) Nucleic acid extraction: a fast DNA extraction reagent kit (TIAGEN, KG203) is used to complete the extraction and purification of the sample's genome DNA; for the detailed operation, please refer to the operation manual of the kit.
(2) Construction of library: the library is constructed by using the general library construction reagent kit (VAHTS Universal Plus DNA Library Prep Kit for Illumina, (Product No.: ND617-02)). For the detailed operation, please refer to the operational manual of the kit.
(3) Quality control of the library: concentration testing and quality control for the length of the segment are conducted for the enriched library.

After the above operations, a library sample of about 50nM having a length of about 1-1000 bp is obtained.

### 2. Preparation of the sequencing flowcell

Illumina's MiSeq sequencing system and its complementary sequencing reagent kit (MiSeq Reagent kit v3) are used to perform library denaturation, library loading and surface amplification to obtain the DNA amplification cluster. Meanwhile the sequencing primer ACACTCTTTCCCTACACGACGCTCTTCCGATC (SEQ ID No. 1) is added. After the hybridization is completed, the flowcell is fixed therein with the DNA amplification cluster which is hybridized with the sequencing primer to wait for the subsequent sequencing reaction.

### 3. Sequencing

### (1) Preparation of the sequencing reagent

Incorporation reaction solution 1: 50 mM Tris-HCL, 50 mM NaCl, 10 mM (NH₄)₂SO₄, 0.02 mg/ml polymerase 9N (Salus-bio), 3 mM MgSO₄, 1 mM EDTA, and the blocker group and the fluorescence group-carrying first nucleotide analogs dATP, dCTP, dGTP and dTTP each being 1 µM;
Incorporation reaction solution 2: 50 mM Tris-HCl, 50 mM NaCl, 10 mM (NH₄)₂SO₄, 0.02 mg/ml polymerase 9N (Salus-bio), 3 mM MgSO₄, 3 mM CuCl₂, and the blocker group only-carrying second nucleotide analogs dATP, dCTP, dGTP and dTTP each being 1 µM;
Elution buffer: 5×SSC, Tween-20 0.05%;
Pre-wash buffer: 50 mM Tris-HCl, 0.5 mM NaCl, 10 mM EDTA, Tween-20 0.05%;
Cleavage reaction solution: 20 mM THPP, 0.5 M NaCl, 50 mM Tris-HCl, pH 9.0, Tween-20 0.05%.

For the blocker group and the fluorescence group-carrying first nucleotide analogs dATP, dCTP, dGTP and dTTP (or the blocker group only-carrying second nucleotide analogs dATP, dCTP, dGTP and dTTP), the dCTP is taken as an example, which is shown below. The blocker group is an azide group, and the fluorescence groups of the dATP, dCTP, dGTP and dTTP respectively are Cy5, ROX, AF-532, IF-700:

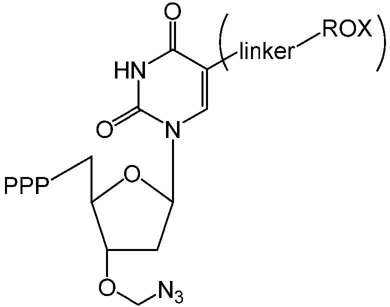

### (2) Sequencing reaction cycle

(a) Incorporation reaction: 200µl of the pre-wash buffer is pumped by an injection pump at a speed of 800µl/min into the flowcell on which the amplification has been performed. Then 200µl of the incorporation reaction solution 1 is pumped by the injection pump at a speed of 800µl/min into flowcell. The temperature is set at 55°C for reaction for 60s. Thereafter, the incorporation reaction solution 2 is pumped by the injection pump at a speed of 800µl/min into the flowcell, and the temperature is set at 50°C.
(b) Signal scanning: when the incorporation reaction solution 2 has totally been pumped into the flowcell, as soon as the reaction occurs, the signal scanning of the entire flowcell is conducted, thereby to identify the type of the base bound to the primer strand on each amplification cluster.
(c) Cleavage reaction: after completion of the signal scanning, the pump first pumps in 200µl of the elution buffer at a speed of 800µl/min, and then the pump pumps 200µl of the cleavage reaction solution at a speed of 800µl/min. The temperature is set at 60°C for reaction for 90s. Thereafter, 200µl of the elution buffer is pumped in at the same speed again to repeat the cleaning process.

Steps (a)-(c) are repeated for a next sequencing cycle. The sequencing cycle is repeated for a total of 100 times.

A normal process of a comparison example 1 includes the following specific steps:
(a) Incorporation reaction: 200µl of the pre-wash buffer is pumped by an injection pump at a speed of 800µl/min into the flowcell on which the amplification has been performed. Then 200µl of the incorporation reaction solution 1 is pumped by the injection pump at a speed of 800µl/min into the flowcell. The temperature is set at 55°C for reaction for 60s. Thereafter, 200µl of the incorporation reaction solution 2 is pumped by the injection pump at a speed of 800µl/min into the flowcell. The temperature is set at 50°C for reaction for 90s.
(b) Signal scanning: after the reaction of the incorporation reaction solution 2 is completed, signal scanning of the entire sequencing flowcell is conducted to identify the type of the base bound to the primer strand on each amplification cluster.
(c) Cleavage reaction: after completion of the signal scanning, the pump first pumps in 200µl of the elution buffer at a speed of 800µl/min, and then the pump pumps in 200µl of the cleavage reaction solution at a speed of 800µl/min. The temperature is set at 60°C for reaction for 90s. Thereafter, 200µl of the elution buffer is pumped in at the same speed again to repeat the cleaning process.

Steps (a)-(c) are repeated for a next sequencing cycle. The sequencing cycle is totally repeated for 100 times.

For the above reaction process, further explanations are given below:
In the step (a) of incorporation reaction, the pre-wash buffer is first pumped in to wash away the possible residue of the reagent used in the previous amplification and hybridization process. The dNTPs carrying both the blocker groups and the fluorescence groups in the incorporation reaction solution 1 which is later pumped in are combined to the primer strands under the action of the polymerase and according to the principle of complementary pairing. Since the existence of the azide groups, the incorporation is blocked so that only one dNTP is bound. The dNTPs carrying only the blocker groups in the incorporation reaction solution 2 which is later pumped in are bound to the primer strands in a similar manner.
In the step (b) of signal scanning, a laser emits excitation light into the flowcell, thereby to excite the fluorescence groups modified by the dNTPs bound to the primer strand to generate a fluorescent light indicative of the base type. By identifying a fluorescence image signal through an optical imaging system, the type of the bound base is obtained.
In the step (c) of cleavage process, the elution buffer is used to wash away the reagent used in the previous steps to create a suitable chemical reaction condition for the cleavage reaction. The cleavage reaction solution is used to remove the blocker groups and the fluorescence groups from the dNTPs carrying both the blocker groups and the fluorescence groups. Thus, the hydroxyl group of the 3' end is exposed for the start of the next cycle.

### 3. Sequencing results

The sequencing results are shown in FIG. 2. According to A of FIG. 2, in each cycle, the period of time for acquiring the signal (period of time for photo taking) is fixed. Since the period of time for reaction of the incorporation reaction solution 1 is reduced, and at the same time, the period of time for reaction of the incorporation reaction solution 2 is integrated into the process of signal scanning. The period time for a single cycle (exclusive of the period of time for signal scanning) is reduced from 360s to 240s, which amounts to a reduction of about 30%. From B of Fig. 2, it can be seen that the sequencing quality Q30 resulted from the improved process basically is the same as the quality of the normal process of the comparison example 1. It indicates that the improved sequencing technology provided by the illustrated embodiment 1 can significantly reduce the sequencing period of time while ensuring the sequencing quality.

### Embodiment 2

This embodiment provides a sequencing method for human genome, including the following steps:

### 1. Construction of sequencing library

(1) Nucleic acid extraction: a fast DNA extraction reagent kit (TIAGEN, KG203) is used to complete the extraction and purification of the sample's genome DNA; for the detailed operation, please refer to the operation manual of the kit.
(2) Construction of library: the library is constructed by using the general library construction reagent kit (VAHTS Universal Plus DNA Library Prep Kit for Illumina (Product No.: ND617-02)). For the detailed operation, please refer to the operational manual of the kit.
(3) Quality control of the library: concentration testing and quality control for the length of the segment are conducted for the enriched library.

After the above operations, a library sample of about 50nM having a length of about 1-1000 bp is obtained.

### 2. Preparation of the sequencing flowcell

Illumina's MiSeq sequencing system and its complementary sequencing reagent kit (MiSeq Reagent kit v3) are used to perform the library denaturation, library loading and surface amplification to obtain the DNA amplification cluster. Meanwhile the sequencing primer ACACTCTTTCCCTACACGACGCTCTTCCGATC is added. After the hybridization is completed, the flowcell is fixed therein with the DNA amplification cluster which is hybridized with the sequencing primer to wait for the subsequent sequencing reaction.

### 3. Sequencing

### (1) Preparation of the sequencing reagent

Incorporation reaction solution 1: 50 mM Tris-HCL, 50 mM NaCl, 10 mM (NH₄)₂SO₄ , 0.02 mg/ml polymerase 9N (Salus-bio), 3 mM MgSO₄, 1 mM EDTA, and the blocker group and the fluorescence group-carrying first nucleotide analogs dATP, dCTP, dGTP and dTTP each being 1 µM;
Incorporation reaction solution 2: 50 mM Tris-HCl, 50 mM NaCl, 10 mM (NH₄)₂SO₄, 0.02 mg/ml polymerase 9N (Salus-bio), 3 mM MgSO₄, 1 mM EDTA, and the blocker group only-carrying second nucleotide analogs dATP, dCTP, dGTP and dTTP each being 1 µM;
Elution buffer: 5×SSC, Tween-20 0.05%;
Pre-wash buffer: 50 mM Tris-HCl, 0.5 mM NaCl, 10 mM EDTA, Tween-20 0.05%;
Cleavage reaction solution: 20 mM THPP, 0.5 M NaCl, 50 mM Tris-HCl, pH 9.0, Tween-20 0.05%.

For the blocker group and the fluorescence group-carrying (or the blocker group only-carrying) dATP, dCTP, dGTP and dTTP, they are the same as those of the embodiment 1.

### 2) Sequencing reaction cycle

(a) Incorporation reaction: 200µl of the pre-wash buffer is pumped by an injection pump at a speed of 1200µl/min into the flowcell on which the amplification has been performed. Then, 200µl of the incorporation reaction solution 1 is pumped by the injection pump at a speed of 1200µl/min into the flowcell. The temperature is set at 55°C.
(b) Signal scanning: when the incorporation reaction solution has totally been pumped into the flowcell, as soon as the reaction occurs, the signal scanning of the entire flowcell is conducted, thereby to identify the type of the base bound to the primer strand on each amplification cluster. At the same time, 200µl of the incorporation reaction solution 2 is pumped in at a speed of 400µl/min, with the temperature being kept at 55°C for reaction for 2 min. After 2min, the pump pumps in 200µl of the elution buffer at a speed of 300µl/min. When the pump completes the pumping of the liquid, the process of the signal scanning exactly ends (or is about to end).
(c) Cleavage reaction: after completion of the signal scanning, the pump pumps in 200µl of the cleavage reaction solution at a speed of 1200µl/min. The temperature is set at 55°C for reaction for 50s. Thereafter, 200µl of the elution buffer is pumped in by the pump at a speed of 1200µl/min.

Steps (a)-(c) are repeated for a next sequencing cycle. The sequencing cycle is totally repeated for 100 times.

Comparison example 2 is similar to the comparison example 1, with a specific photo-taking process used for the signal scanning thereof being the same as that of the embodiment 2, but different from that of the embodiment 1 and the comparison example 1.

### 3. Sequencing results

The sequencing results are shown in FIG. 3. According to A of FIG. 3, under the process which is optimized as much as possible, except the period of time for the signal scanning which is fixed, the sequencing process proposed by the embodiment 2 can reduce the period of time for each cycle to 90s, which amounts to a reduction of 75% as compared to 360s of the normal process of the comparison example 2. The signal scanning (photo-taking process) adopted by the comparison example 2 is different from that adopted by the comparison example 1 of the embodiment 1, resulting in a system error, whereby the experiment results of the sequencing qualities Q30 of the two comparison examples are not consistent. In comparison of the sequencing qualities between the comparison example 2 and the embodiment 2, although the value of the sequencing quality Q30 of the latter is lower, it does not affect the entire sequencing quality too much. Thus, the total period of time for the sequencing can be largely reduced by integrating the process of injecting the incorporation reaction solution 2 and the elution buffer by controlling the pumping speed of the incorporation reaction solution 2 and the pumping speed of the elution buffer to match with the progress of the signal scanning.

### Embodiment 3

This embodiment provides a sequencing method, including the following steps:

### 1. Construction of sequencing library

(1) Nucleic acid extraction: a fast DNA extraction reagent kit (TIAGEN, KG203) is used to complete the extraction and purification of the sample's genome DNA; for the detailed operation, please refer to the operation manual of the kit.
(2) Construction of library: the library is constructed by using the general library construction reagent kit (VAHTS Universal Plus DNA Library Prep Kit for Illumina (Product No.: ND617-02)). For the detailed operation, please refer to the operational manual of the kit.
(3) Quality control of the library: concentration testing and quality control for the length of the segment are conducted for the enriched library.

After the above operations, a library sample of about 50nM having a length of about 1-1000 bp is obtained.

### 2. Preparation of the sequencing flowcell

Illumina's MiSeq sequencing system and its complementary sequencing reagent kit (MiSeq Reagent kit v3) are used to perform the library denaturation, library loading and surface amplification to obtain the DNA amplification cluster. Meanwhile the sequencing primer ACACTCTTTCCCTACACGACGCTCTTCCGATC is added. After the hybridization is completed, the flowcell is fixed therein with the DNA amplification cluster which is hybridized with the sequencing primer to wait for the subsequent sequencing reaction.

### 3. Sequencing

### (1) Preparation of the sequencing reagent

Incorporation reaction solution 1: 50 mM Tris-HCL, 50 mM NaCl, 10 mM (NH₄)₂SO₄, 0.02 mg/ml polymerase 9N (Salus-bio), 3 mM MgSO₄, 1 mM EDTA, and the blocker group only-carrying dATP, dCTP, dGTP and dTTP (second nucleotide analogs) each being 1 µM;
Incorporation reaction solution 2: 50 mM Tris-HCl, 50 mM NaCl, 10 mM (NH₄)₂SO₄, 0.02 mg/ml polymerase 9N (Salus-bio), 3 mM MgSO₄, 3 mM CuCl₂, and the fluorescence group only-carrying dATP, dCTP, dGTP and dTTP (third nucleotide analogs) each being 1 µM;
Elution buffer: 5×SSC , Tween-20 0.05% ;
Pre-wash buffer: 50 mM Tris-HCl, 0.5 mM NaCl, 10 mM EDTA, Tween-20 0.05% ;
Cleavage reaction solution: 20 mM THPP, 0.5 M NaCl, 50 mM Tris-HCl, pH 9.0, Tween-20 0.05%.

Imaging reaction solution: 1 mM Tris-HCL, 40 mM Sodium L-Ascorbate, 50mM NaCl, Tween-20 0.05%.

For the blocker group only-carrying or the fluorescence group only-carrying dATP, dCTP, dGTP and dTTP, please refer to the embodiment 1.

### (2) Sequencing reaction cycle

(a) Incorporation reaction: 200µl of the pre-wash buffer is pumped by an injection pump at a speed of 800µl/min into the flowcell on which the amplification has been performed. Then, 400µl of the incorporation reaction solution 1 is pumped by the injection pump at a speed of 800µl/min into the flowcell, and the temperature is set at 55°C for reaction for 20s. Then, 200µl of the elution buffer is pumped into the flowcell at a speed of 800µl/min, in order to wash away the second nucleotide analogs which have not been completely reacted. Thereafter, 400µl of the incorporation reaction solution 2 is pumped by the injection pump at a speed of 800µl/min into the flowcell, and the temperature is set at 55°C for reaction for 70s, whereby under the action of the polymerase 9N and the metal ions Mg²⁺ and Cu2⁺, the incorporation reaction solution 2, the nuclei acid molecular to be detected and the second nucleotide analogs form a stable complex, such that the third nucleotide analogs are stably chelated at the position of a next nucleotide of the incorporation reaction of the previous step. Thereafter, the injection pump pumps in 200µl of the elution buffer at a speed of 800µl/min to wash away the third nucleotide analogs which have not been bound.
(b) Signal scanning: as soon as the incorporation reaction solution 2 has been totally pumped in, the signal scanning of the entire chip is conducted, thereby to identify the type of base bound to the primer strand on each amplification cluster.
(c) Cleavage reaction: after completion of the signal scanning, the injection pump pumps in 600µl of the pre-wash buffer at a speed of 900µl/min, and the reaction lasts for 1min. Thereafter, the injection pump pumps in 200µl of the cleavage reaction solution at a speed of 800µl/min, and the temperature is set at 60°C for reaction for 30s, and 200µl of the elution buffer is then pumped in at the same speed.

Steps (a)-(c) are repeated for a next sequencing cycle. The sequencing cycle is totally repeated for 100 times.

The comparison example 3 is only different from the embodiment 3 in the steps of the sequencing reaction cycle. Specific details are given as the following:
In the embodiment 3, the step of the signal scanning is started as soon as the pumping in of the incorporation reaction solution 2 containing the third nucleotide analogs has been completed. And the third nucleotide analogs which have not been bound are washed completely before the end of the signal scanning step, while the comparison example 3 starts the signal scanning after the end of the reaction of the incorporation reaction solution 2 and the end of the pumping in of the elution buffer and the elution process.

As shown in FIG. 4, in comparison with the comparison example 3 which starts the signal scanning after the completion of the reaction of the incorporation reaction solution 2, and pumps in the pre-wash buffer after the end of the signal scanning, this embodiment starts the signal scanning after the beginning of the reaction of the incorporation reaction solution 2. Meanwhile, the pre-wash buffer is pumped in during the signal scanning step where a portion of the signal scanning has been completed, which optimizes the operation process, and reduces the period of time of the sequencing (not including the photo-taking), without obviously lowering the quality of the sequencing.

As can be seen from the above embodiments, the sequencing method proposed by embodiments of the present application is not limited to any particular next-generation sequencing method. All next-generation sequencing technologies based on SBS (sequencing by synthesis) can use the above methods to optimize the sequencing.

The above is a detailed explanation for the embodiments of the present application. However, the present application is not limited to the above-mentioned embodiments. To those of ordinary skill in the art, various modifications could be made without departing from the principle of the present application. In addition, under the condition of no conflict, the embodiments and the features of the embodiments of the present application can be combined together.

## Claims

1. A sequencing method, which is **characterized by** comprising following steps:
incorporation: combining a detection complex to a primer hybridized to a nucleic acid molecule to be detected, wherein the detection complex is modified with a fluorescence group and a blocker group;
scanning: exciting the fluorescence group to generate a fluorescence signal, and identifying the fluorescence signal to acquire a type of base;
cleavage: removing the fluorescence group and the blocker group;
repeating a cycle of the incorporation-scanning-cleavage;
wherein the scanning is started after the beginning of the incorporation step, and/or the cleavage is started after the beginning of the scanning step.

2. The sequencing method of claim 1, **characterized in that** the step of combining a detection complex to a primer hybridized to a nucleic acid molecule to be detected, comprises:
feeding the detection complex and a polymerase onto a surface of a solid phase carrier, the surface being loaded with the nucleic acid molecule to be detected, and the primer being hybridized to the nucleic acid molecule to be detected; and
under the action of the polymerase, the detection complex being combined to the primer and a blockage occurring.

3. The sequencing method of claim 1, **characterized in that** the detection complex includes a first nucleotide analog, and the step of combining a detection complex to a primer hybridized to a nucleic acid molecule to be detected comprises:
feeding the first nucleotide analog and the polymerase onto the surface of the solid phase carrier; and
under the action of the polymerase, the first nucleotide analog being combined to the primer and a blockage occurring to thereby form the detection complex.

4. The sequencing method of claim 3, **characterized in that** the scanning is started after the beginning of the step of feeding the first nucleotide analog and the polymerase onto the surface of the solid phase carrier.

5. The sequencing method of claim 3, **characterized in that**, in the step of incorporation, after the formation of the detection complex, the step further comprises:
feeding a second nucleotide analog and the polymerase onto the surface of the solid phase carrier; and
under the action of the polymerase, the second nucleotide analog being combined to the primer which has not been combined with the first nucleotide analog, and a blockage occurring.

6. The sequencing method of claim 5, **characterized in that** the scanning is started after the beginning of the step of feeding the second nucleotide analog and the polymerase onto the surface of the solid phase carrier.

7. The sequencing method of claim 5, **characterized in that** the second nucleotide analog is modified with a blocker group.

8. The sequencing method of claim 5, **characterized in that** the first nucleotide analog is modified with a fluorescence group and a blocker group.

9. The sequencing method of claim 2, **characterized in that** the solid phase carrier is a flowcell.

10. The sequencing method of claim 2, **characterized in that** removing the blocker group and the fluorescence group comprises:
feeding cleavage reagent onto the surface of the solid phase carrier to remove the blocker group and the fluorescence group in the detection complex.

11. The sequencing method of claim 10, **characterized in that**, before the cleavage, an elution reagent is fed onto the surface of the solid phase carrier to remove residue reagent on the surface.

12. The sequencing method of claim 10, **characterized in that**, the step of feeding the elution reagent onto the surface of the solid phase carrier to remove residue reagent on the surface is started after the beginning of the step of scanning.

13. The sequencing method of claim 1, **characterized in that**, the start of the scanning after the beginning of the step of incorporation comprises starting the scanning after 0-60 seconds of the beginning of the step of incorporation.

14. The sequencing method of claim 1, **characterized in that**, the start of the cleavage after the beginning of the step of scanning comprises starting the cleavage after 0-10 minutes of the beginning of the step of scanning.
